(19) Europäisches Patentamt
European Patent Office
Office européen des brevets

(11) Veröffentlichungsnummer: **0 133 493**
**B1**

(12) **EUROPÄISCHE PATENTSCHRIFT**

(45) Veröffentlichungstag der Patentschrift: **25.01.89**

(51) Int. Cl.⁴: **C 07 D 307/62**

(21) Anmeldenummer: **84108532.7**

(22) Anmeldetag: **19.07.84**

(54) **Verfahren zur Herstellung von Kohlenhydraten.**

(30) Priorität: **05.08.83 CH 4268/83**

(43) Veröffentlichungstag der Anmeldung:
**27.02.85 Patentblatt 85/9**

(45) Bekanntmachung des Hinweises auf die Patenterteilung:
**25.01.89 Patentblatt 89/4**

(84) Benannte Vertragsstaaten:
**AT BE CH DE FR GB IT LI NL**

(56) Entgegenhaltungen:
**EP-A-0 086 324**
**CH-A-256 514**
**DE-C-854 514**
**DE-C-892 446**
**DE-C-1 142 861**

(73) Patentinhaber: **F. HOFFMANN- LA ROCHE & CO. Aktiengesellschaft, CH- 4002 Basel (CH)**

(72) Erfinder: **Fahrni, Peter, Dr., Obere Hofackerstrasse 23, CH- 4414 Füllinsdorf (CH)**
Erfinder: **Siegfried, Theodor, Dr., Türkheimerstrasse 5, CH- 4009 Basel (CH)**

(74) Vertreter: **Cottong, Norbert A., Grenzacherstrasse 124 Postfach 3255, CH- 4002 Basel (CH)**

**EP 0 133 493 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1989

**Beschreibung**

Die vorliegende Erfindung betrifft ein neues Verfahren zur Herstellung von Kohlenhydraten und insbesondere von Ascorbinsäure, Isoascorbinsäure und 5-Ketoascorbinsäure ausgehend von 2-Ketogalactonsäureestern, 2-Ketogluconsäureestern bzw. 2,5-Diketogluconsäureestern.

Isoascorbinsäure ist ein bekanntes Antioxidans und 5-Ketoascorbinsäure ist ein bekanntes Zwischenprodukt zur Herstellung von Ascorbinsäure.

Das erfindungsgemässe Verfahren zur Herstellung der vorhergehend erwähnten Säuren der allgemeinen Formel

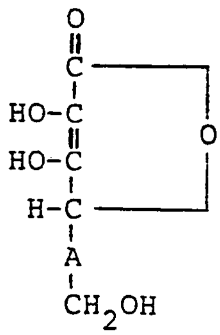

I

worin A die Gruppe

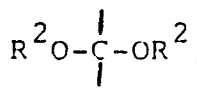

,

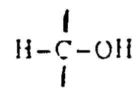

oder

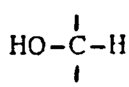

darstellt und $R^2$ Alkyl mit 1 bis 5 Kohlenstoffatomen bedeutet,

ist dadurch gekennzeichnet, dass man eine Verbindung der allgemeinen Formel

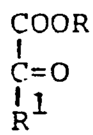

II

worin R Alkyl mit 1 bis 5 Kohlenstoffatomen, $R^1$ eine Gruppe der Formel

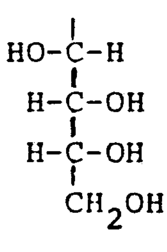

,

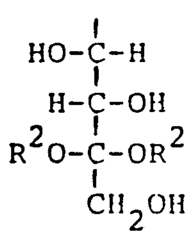

oder

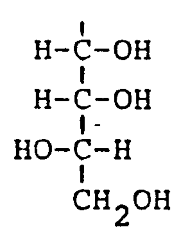

darstellen und $R^2$ obige Bedeutung hat,

mit einem Amin mit 12 bis 38 Kohlenstoffatomen in einem organischen Lösungsmittel, wie unlen definiert, umsetzt, dass man das erhaltene Aminsalz der allgemeinen Formel

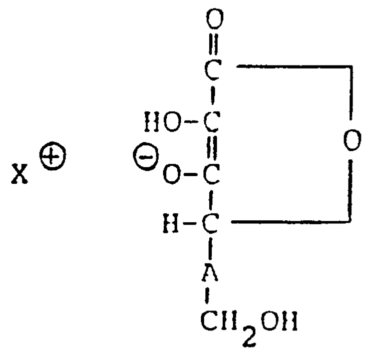 III

worin A die obige Bedeutung hat und X ⊕ das Ammoniumion des eingesetzten Amins darstellt,

ohne Neutralisation, wie unten angegeben, spaltet und die Verbindung der Formel I sowie gegebenenfalls das eingesetzte Amin isoliert.

Es sind bereits Verfahren zur Herstellung von Ascorbinsäure durch Lactonisierung bekannt geworden:

So beschreiben:

**EP-A 0086 324**

Ascorbinsäure durch Umlagerung von Ketogulonsäureestern in alkalischem Milien, ebenfalls mittels $C_{12-38}$-Aminen. Spaltung des Ascorbinsäureaminsalzes ohne Neutralisiation (das Zitat fällt unter Art. 54 (3) EPÜ).

**CH-A-256 5124**

Lactonisierung von niederen aliphatischen Estetn der 2-Ketogulonsäure mittels wasserfreiem Ammoniak in einem wasserfreien niederen aliphatischen Alkohol, via Amid und Ammoniumsalz der Ascorbinsäure.

**DE-C-892 446**

Konversion von Ketogulonsäureestern in alkoholischer Lösung mittels Aminsalzen schwacher Säuren; die Aminogruppen tragen kurze C-Ketten.

Der Ausdruck "Alkyl" bedeutet im Rahmen der vorliegenden Erfindung Alkylgruppen mit 1 bis 5 Kohlenstoffatomen wie Melhyl, Äthyl, Propyl, Isopropyl, Butyl.

Bevorzugt sind Alkylgruppen mit 1 bis 3 Kohlenstoffatomen und insbesondere Methyl und Äthyl.

Als Amine kommen im Rahmen der vorliegenden Erfindung primäre, sekundäre und tertiäre Amine mit 12 bis 38 Kohlenstoffatomen in Betracht. Die untere Grenze der Kohlenstoffanzahl ist u.a. von der Löslichkeit in dem zur Spaltung des Aminsalzes der Formel III verwendeten organischen Lösungsmittel abhängig. Die Obergrenze der Kohlenstoffatomanzahl wiederum ist abhängig von der Löslichkeit in dem für die Reaktion zwischen dem Ester der Formel II und dem Amin verwendeten Lösungsmittel.

Die verwendbaren Amine sind in der Reihenfolge bevorzugt:

tert. Amine > sek. Amine > prim. Amine

Primäre Amine umfassen sowohl geradkettige, als auch verzweigte Amine, wobei die verzweigten bevorzugt sind. Besonders bevorzugt sind zudem solche mit 12 bis 24 Kohlenstoffatomen. Unter primären Aminen sind zudem gewisse flüssige, basische Ionentauscher wie etwa Primene JMT® oder auch Amberlite LA-3® zu verstehen.

Sekundäre Amine umfassen sowohl geradkettige, als auch verzweigte Alkylamine, sowie aromatische Amine. Bevorzugte sekundäre Amine sind verzweigte Alkylamine und insbesondere solche mit 16 bis 25 Kohlenstoffatomen. Von den aromatischen sekundären Aminen ist das Dibenzylamin bevorzugt. Unter sekundären Aminen sind zudem gewisse flüssige, basische Ionentausche wie etwa Amberlite LA-1® oder Amberlite LA-2® zu verstehen.

Terhiäre Amine umfassen insbesonere geradkettige und teilweise verzweigte aliphatische Amine, wobei die geradkettigen Alkylamine und insbesondere solche mit 15 bis 30 Kohlenstoffatomen bevorzugt sind.

Sowohl bei den sekundären, als auch bei den tertiären Aminen kommen sowohl solche mit gleichen, als auch solche mit verschiedenen Alkylketten in Frage.

Als Beispiele von im Rahmen der vorliegenden Erfindung in Frage kommenden und zudem, bevorzugten Aminen können folgende genannt werden:

| | |
|---|---|
| Primene JMT® | Trihexylamin |
| Amberlite LA-3® | Triheptylamin |
| Bis-(2-äthylhexyl)amin | Trioctylamin |
| Amberlite LA-1® | Tridodecylamin |
| Amberlite LA-2® | |
| Dioctylamin | |
| Tripentylamin | |
| Triisopentylamin | |
| N,N-Dioctylmethylamin | |

Die Umsetzung der Ester der Formel II, welche im übrigen auch in pyranoider oder furanoider Form vorliegen können, mit einem Amin erfolgt in einem organischen Lösungsmittel, bzw. in einem Lösungsmittelgemisch, in welchem diese Ester und/oder das verwendete Amin zumindest teilweise löslich sind. In Frage kommen hierbei sowohl

protische, als auch

aprotische-dipolare Lösungsmittel. Von den protischen können beispielsweise genannt werden niedere Alkohole mit 1 bis 5 Kohlenstoffatomen, wie Methanol, Äthanol, Propanol, Isopropanol usw. Als Beispiele von aprotisch-dipolaren Lösungsmitteln können genannt werden Acetonitril, Dimethylformamid, Dioxan, Monoglyme, Methylcellosolve (Älhylenglycolmonomethyläther). Bevorzugte Lösungsmittel sind die Alkohole und insbesondere Methanol.

Die als Ausgangsmaterial verwendeten Ester der Formel II sind bekannte oder Analoge bekannter Verbindungen. Sie können als solche in der Reaktion eingesetzt werden oder auch vorgängig in situ aus den entsprechenden Säuren durch Veresterung mit einem entsprechenden Alkohol hergestellt werden.

Die verwendete Menge an Lösungsmittel ist an und für sich nicht kritisch, liegt aber zweckmässig bei einem Verhältnis von etwa 1 : 1 bis etwa 10 : 1 (ml/g), bezogen auf den Ester und das eingesetzte Amin.

Die in dem erfindungsgemässen Verfahren verwendete Menge Amin beträgt zweckmässig von 0,1 bis 1,5, vorzugsweise von 0,5 bis 1,1 und insbesondere von etwa 0,9 bis 1 Mol pro Mol Ester.

Die Temperatur, der Druck und die Reaktionsdauer sind als solche keine kritischen Grössen in dem erfindungsgemässen Verfahren.

Die Temperatur ist der geschwindigkeits-bestimmende Faktor. Die obere Grenze ist dabei durch die Stabilität der Reaktionspartner gegeben. Zweckmässig erfolgt die Reaktion bei einer Temperatur bis 90°C, vorzugsweise von 50°C bis 75°C und insbesondere bei etwa 65°C.

Der Druck ist bei dem erfindungsgemässen Verfahren keine kritische Grösse, so dass dieses ohne weiteres bei Normaldruck durchgeführt werden kann. Es kann jedoch u.U. auch unter erhöhtem Druck gearbeitet werden, bei entsprechend erhöhter Temperatur und entsprechend geringeren Reaktionszeiten.

Die Reaktionszeit ist abhängig von der Reaktionstemperatur und liegt im Hinblick auf die optimalen Temperaturen zwischen 2 bis 7 und insbesondere zwischen 3 bis 5 Stunden.

Die Umsetzung des Esters und des Amins kann sowohl in Anwesenheit, als auch in Abwesenheih von Luft durchgeführt werden. Vorzugsweise wird allerdings unter Luftausschluss, d.h. unter Inertgas, wie etwa Slickstoff, Argon gearbeitet.

Die Spaltung des erhaltenen Aminsalzes der Formel III erfolgt erfindungsgemäss ohne Neutralisation, d.h. ohne Zugabe einer Säure oder einer Base. Die Spaltung, sowie die Isolierung der reinen Verbindungen der Formel I und gegebenenfalls des eingesetzten Amins, erfolgt durch flüssig-flüssig Extraktion, wobei die Verbindungen der Formel I in die polare und das freie Amin in die unpolare Phase übergehen, oder u.U.

durch blosses Digerieren, d.h. Erhitzen mit einem geeigneten, d.h. unpolaren organischen Lösungsmittel.

Für die Extraktion geeignete unpolare Lösungsmittel sind solche, in denen das verwendete Amin gut löslich ist. Zweckmässig verwendet man ein aprotisch apolares Lösungsmittel, wie aliphatische oder aromatische Kohlenwasserstoffe, z. B. Hexan, Petroläther. Benzol, Toluol, Xylol.

Geeignete polare Lösungsmittel bzw. Lösungsmittelgemische sind solche, in denen die Verbindungen der Formel I gut löslich sind und welche mit den vorhergehend erwähnten unpolaren Lösungsmitteln nicht mischbar sind. Zweckmässig verwendet man Wasser, Methanol, Äthanol, Acetonitril, Aceton, bzw. Gemische hiervon. Als besonders zweckmässig hat sich die Anwesenheit zumindest einer geringen Menge Wasser erwiesen. Besonders bevorzugt ist Wasser oder ein Wasser-Methanol-Gemisch.

Die Temperatur, bei welcher die Extraktion durchgeführt wird, ist an und für sich nicht kritisch, und es kann sowohl bei Raumtemperatur, als auch bei erhöhter Temperatur gearbeitet werden. Vorzugsweise wird die Extraktion bei etwa Raumtemperatur durchgeführt. Nach erfolgter Extraktion können die Verbindungen der Formel I sowie das verwendete Amin aus den jeweiligen sie enthaltenden Phasen in an sich bekannter Weise leicht isoliert werden.

Für das Digerieren sind im Prinzip die gleichen unpolaren Lösungsmittel geeignet wie vorhergehend für die Extraktion angegeben. Das Erhitzen erfolgt zweckmässig bei einer Temperatur von etwa 50°C bis Rückflusstemperatur des verwendeten Lösungsmittels. Vorzugsweise erfolgt das Digerieren bei Rückflusstemperatur. Bei diesem Digerieren wird das Aminsalz der Formel III zerlegt, wobei die Verbindungen der Formel I ausfallen und das Amin in die organische Phase übergeht, aus welcher es dann leicht in an sich bekannter Weise isoliert werden kann.

Sämtliche vorhergehend erwähnten Operationen können im übrigen sowohl kontinuierlich, als auch batchwise durchgeführt werden.

Die nachfolgenden Beispiele sollen die Erfindung weiter exemplifizieren, ohne jedoch in irgendeiner Weise eine Beschränkung darzustellen.

**Beispiel 1**

In einen 100-ml-Sulfierkolben, versehen mit Rührer, Thermometer, Rückflusskühler und 10-ml-Tropftrichter, werden unter Argon 4,16 g (20,0 mMol) 2-Ketogluconsäuremethylester und 19,4 ml Methanol gegeben. Hierauf wird das Gemisch unter Rühren auf 65° C (Rückfluss) erwärmt.

Mittels des Tropftrichters werden dann 5,39 g (100 Mol %) Trihexylamin (99,9-%-ig) innert 15 Minuten zugetropft. Nun wird während 4 3/4 Stunden bei Rückfluss weitergerührt, wobei sich das Reaktionsgemisch gelb färbt. Nach insgesamt 5 Stunden wird das Gemisch mit Methanol in einen 250-ml-Rundkolben gespült und am Rollverdampfer bei 45° C 20 mbar eingeengt. Als Rückstand erhält man ein gelbes, viskoses Öl.

Das vorhergehend erhaltene Öl wird mit 50 ml entionisiertem Wasser in einen mit Stickstoff begasten Rotations-Perforator gespült. Hierauf wird das Gemisch mit etwa 500 ml n-Hexan während 20 Stunden kontinuierlich extrahiert. Nach beendeter Extraktion werden die beiden im Extraktor verbleibenden Phasen in einem Scheidetrichter getrennt. Die organischen Phasen werden vereinigt, über etwa 30 g $Na_2SO_4$ getrocknet und am Rollverdampfer bei 45° C/100 - 20 mbar eingedampft. Zurück bleiben 5,48 g gelbes Trihexylamin mit einem Gehalt von 96,6 %. Dies entspricht einer Ausbeute an Trihexylamin von 98,2 %.

Die nach der Extraktion des Trihexylamins angefallene Wasserphase enthält 3,18 g Isoascorbinsäure, entsprechend einer Ausbeute von 90,4 %. Aus dieser Lösung werden 1,97 g Isoascorbinsäure mit einem Gehalt von 99,1 % kristallisiert.

**Beispiel 2**

In einen 200-ml-Sulfierkolben, versehen mit Rührer, Thermometer, Rückflusskühler und 50 ml Tropftrichter, werden unter Argon 13,4 g (64,4 mMol) 2-Ketogluconsäuremethylester und 63 ml Methanol gegeben. Hierauf wird das Gemisch unter Rühren auf 65° C (Rückfluss) erwärmt. Mittels des Tropftrichters werden dann 22,8 g (100 Mol %) Trioctylamin innert 15 Minuten zugetropft. Nun wird während 4 3/4 Stunden bei Rückfluss weitergerührt, wobei sich das Reaktionsgemisch gelb färbt. Nach insgesamt 5 Stunden wird das Methanol abdestilliert und laufend mit Toluol ersetzt. Nach 1 Stunde werden 80 ml Lösungsmittel ausgetauscht, wobei die Temperatur auf 105° C steigt. Bei 100° C beginnt eine braune Kristallmasse zu kristallisieren. Anschliessend wird noch 1 Stunde bei Rückflusstemperatur weitergerührt und der Niederschlag dann heiss abgenutscht. Der Rückstand wird mit 7 ml Toluol und dann mit 3 x 10 ml Aceton gewaschen und über Nacht bei Raumtemperatur/20 mbar im Vakuumtrockenschrank getrocknet. Man erhält 5,52 g (48,7 %) Isoascorbinsäure mit einem Gehalt von 99,1 %.

Das bei der Filtration anfallende Filtrat wird am Rotationsverdampfer vollständig eingedampft und man erhält 27,3 g (120 % Gewichtsausbeute) rohes Trioctylamin zurück.

**Beispiel 3**

In zu Beispiel 1 analoger Weise wird 2-Ketogluconsäuremethylester mit weiteren Aminen umgelagert. Die Resultate sind in der nachfolgenden Tabelle zusammengestellt.

**Tabelle**

| Amin | Menge Mol % | Lösungsmittel | Extraktionsmittel | % Ausbeute Amin a) | Iso-ASC b) |
|---|---|---|---|---|---|
| Tripentylamin | 100 | Methanol | Hexan | 98,2 | 94,6 |
| Trihexylamin | 100 | Äthanol | Methylcyclohexan | 100 | 94,4 |
| Triheptylamin | 50 | Methanol | Hexan | 99,1 | 89,9 |
| Tridodecylamin | 100 | Methanol | Hexan | 98,0 | 91,6 |
| Primene JMT® c) | 100 | Methanol | Hexan | 73,1 | 86,8 |
| Bis-(2-äthylhexyl)amin | 100 | Methnnol | Petroläther (tiefsiedend) | 99,6 | 95,5 |
| Amberlite LA-2® d) | 100 | Methanol | Petroläther (tiefsiedend) | 91,5 | 96,2 |

a) org. Phase nach Extraktion, eingedampft und titriert
b) wässrige Phase nach Extraktion, Redoxtitration (Primene: HPLC)
c) Extraktionszeit: 68 Stunden
d) flüssiger Ionentauscher der Firma Rohm u. Haas

**Beispiel 4**

In einen 100-ml-Rundkolben, versehen mit Magnetrührer und Rückflusskühler, werden unter Stickstoff-Begasung 5,05 g (20 mMol) 2,5-Diketogluconsäuremethylester-5-dimethylketal und 50 ml getrocknetes Methanol gegeben. Danach werden 6,76 ml (20 mMol) Trihexylamin zugegeben und das Reaktionsgemisch auf Rückflusstemperatur erhitzt, wobei sich der Ester löst. Die Reaktionslösung wird nun 48 Stunden am Rückfluss gerührt. Hierauf wird die Reaktionslösung am Rollverdampfer auf ein Gewicht von 12,1 g eingeengt. Als Rückstand erhält man ein gelbes viskoses Öl. Dieses Öl wird in 25 ml deionisiertem Wasser aufgenommen und in einem Scheidetrichter mit 50 ml Hexan extrahiert. Die Hexanphase wird mit 10 ml Wasser gewaschen und mit ca. 15 g $Na_2SO_4$ getrocknet und anschliessend am Rollverdampfer vollständig eingedampft. Zurück bleiben 4,95 g hellgelbes Amin mit einem Gehalt von 96,2 %. Aminausbeute aus Scheidetrichter Extraktion: 88,4 %. Die Wasserphase wird in einen 100-ml-Kutscher-Steudl-Extraktor gegeben und die Extraktion mit 500 ml Hexan während 16 Stunden fortgeführt. Die fast farblose Hexanphase wird mit ca. 30 g $Na_2SO_4$ getrocknet und eingeengt. Es resultieren 0,68 g Amin mit einem Gehalt von 96,6 %. Aminausbeute aus kontinuierlicher Extraktion: 12,2 %.

Die nach der Extraktion des Trihexylamins angefallene Wasserphase enthält 20,6 % 2,5-Diketogluconsäuremethylester-5,5-dimethylketal und 64,1 % 5-Ketoascorbinsäure-5,5-dimethylketal.

**Beispiel 5**

In zu Belspiel 4 analoger Weise wird 2,5-Diketogluconsäuremethylester-5,5-dimethylketal umgesetzt. Die Resultate sind in folgender Tabelle zusammengefasst.

**Tabelle**

| Amin | Menge Mol% | Amin | Ausbeute % Endprodukt | Ausgangsprod. |
|---|---|---|---|---|
| Amberlite LA-2® | 100 | 89 | 53 a) | 8 a) |
| Trihexylamin | 120 | 95 | 64 b) | 9 b) |

a) Ausbeute in wässriger Lösung
b) Ausbeute als Kristallisat

**Patentansprüche**

1. Verfahren zur Herstellung von Verbindungen der allgemeinen Formel

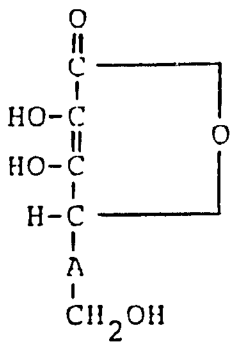

I

worin A die Gruppe

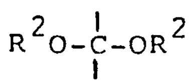
,
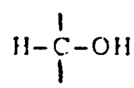

oder

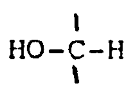

darstellt und $R^2$ Alkyl mit 1 bis 5 Kohlenstoffatomen bedeutet,
dadurch gekennzeichnet, dass man eine Verbindung der allgemeinen Formel

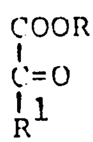

II

worin R Alkyl mit 1 bis 5 Kohlenstoffatomen und $R^1$ eine Gruppe der Formel

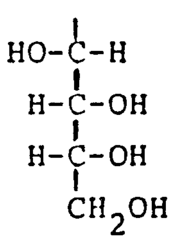
,
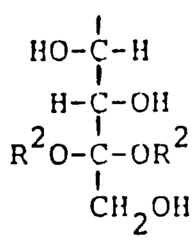
oder
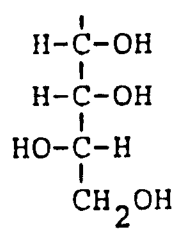

darstellen und $R^2$ obige Bedeutung hat, mit einem Amin mit 12 bis 38 Kohlenstoffatomen in einem protischen oder aprotischen-dipolaren organischen Lösungsmittel bzw. Lösungsmittelgemisch umsetzt, dass man das erhaltene Aminsalz der allgemeinen Formel

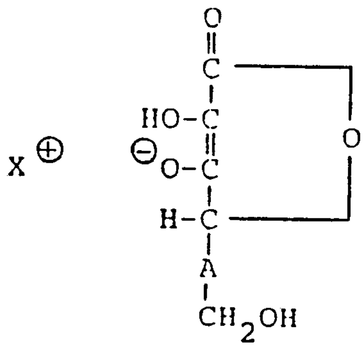

III

worin A die obige Bedeutung hat und X⊕ das Ammoniumion des eingesetzten Amins darstellt, ohne Neutralisation durch flüssig-flüssig Extraktion oder durch Digerieren mit einem unpolaren organischen Lösungsmittel spaltet und die Verbindung der Formel I sowie gegebenenfalls das eingesetzte Amin isoliert.

2. Verfahren nach Anspruch 1, dadurch gekennzeichnet, dass man als Amin ein geradkettiges tertiäres Alkylamin mit 15 bis 30 Kohlenstoffatomen verwendet.

3. Verfahren nach Anspruch 1, dadurch gekennzeichnet, dass man als Amin ein verzweigtes sekundäres Alkylamin mit 16 bis 25 Kohlenstoffatomen verwendet.

4. Verfahren nach Anspruch 1, dadurch gekennzeichnet, dass man als Amin ein verzweigtes primäres Alkylamin mit 12 bis 24 Kohlenstoffatomen verwendet.

5. Verfahren nach Anspruch 1 oder 2, dadurch gekennzeichnet, dass man als Amin Tripentylamin, Triisopentylamin, N,N-Dioctylmethylamin, Trihexylamin, Triheptylamin, Trioctylamin oder Tridodecylamin verwendet.

6. Verfahren nach Anspruch 1 oder 3, dadurch gekennzeichnet, dass man als Amin Bis-(2-äthylhexyl)amin, Dioctylamin, Amberlite LA-1® oder Amberlite LA-2® verwendet.

7. Verfahren nach Anspruch 1 oder 4, dadurch gekennzeichnet, dass man als Amin Primene JMT® oder Amberlite LA-3®, verwendet.

8. Verfahren nach einem der Ansprüche 1 bis 7, dadurch gekennzeichnet, dass das Amin in einer Menge von 0,1 bis 1,5, vorzugsweise von 0,5 bis 1,1 Mol pro Mol Ester verwendet wird.

9. Verfahren nach Anspruch 8, dadurch gekennzeichnet, daß das Amin in einer Menge von 0,9 bis 1 Mol pro Mol Ester verwendet wird.

10. Verfahren nach einem der Ansprüche 1 bis 9, dadurch gekennzeichnet, dass man die Spaltung des Aminsalzes durch flüssig-flüssig Extraktion mittels unpolarer/polarer miteinander nicht-mischbarer Lösungsmittel durchführt.

## Claims

1. A process for the manufacture of compounds of the general formula

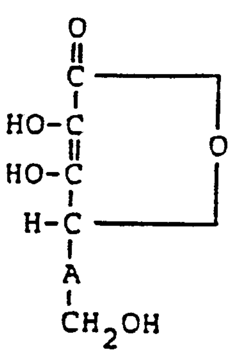

I

wherein A represents the group

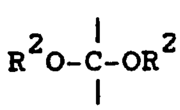, 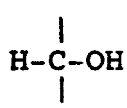

or

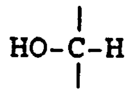

and $R^2$ signifies alkyl with 1 to 5 carbon atoms, characterized by reacting a compound of the general formula

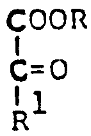

II

wherein R represents alkyl with 1 to 5 carbon atoms and $R^1$ represents a group of the formula

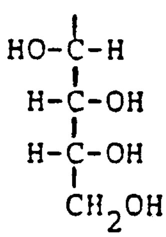 , 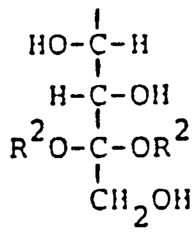 or 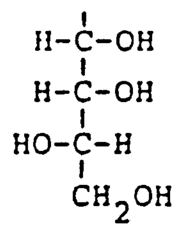

and $R^2$ has the above significance, with an amine with 12 to 38 carbon atoms in a protic or aprotic-dipolar organic solvent or solvent mixture, cleaving the resulting amine salt of the general formula

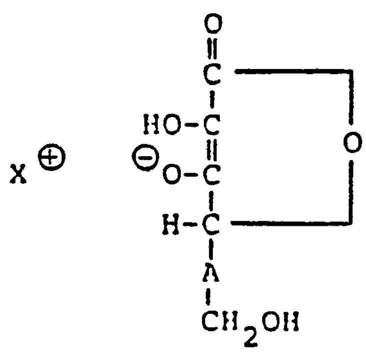

III

wherein A has the above significance and $X^{\oplus}$ represents the ammonium ion of the amine used, without neutralization by liquid-liquid extraction or by digestion with a non-polar organic solvent and isolating the compound of formula I as well as, if desired, the amine used.

2. A process according to claim 1, characterized in that a straight-chain tertiary alkylamine with 15 to 30 carbon atoms is used as the amine.

3. A process according to claim 1, characterized in that a branched secondary alkylamine with 16 to 25 carbon atoms is used as the amine.

4. A process according to claim 1, characterized in that a branched primary alkylamine with 12 to 24 carbon atoms is used as the amine.

5. A process according to claim 1 or 2, characterized in that tripentylamine, triisopentylamine, N,N-dioctylmethylamine, trihexylamine, triheptylamine, trioctylamine or tridodecylamine is used as the amine.

6. A process according to claim 1 or 3 characterized in that bis-(2-ethylhexyl)amine, dioctylamine, Amberlite LA-1® or Amberlite LA-2® is used as the amine.

7. A process according to claim 1 or 4 characterized in that Primene JMT® or Amberlite LA-3® is used as the amine.

8. A process according to any one of claims 1 to 7, characterized in that the amine is used in an amount of 0.1 to 1.5, preferably of 0.5 to 1.1, mol per mol of ester.

9. A process according to claim 8, characterized in that the amine is used in an amount of 0.9 to 1 mol per mol of ester.

10. A process according to any one of claims 1 to 9 characterized in that the cleavage of the amine salt is carried out by liquid-liquid extraction using non-polar/polar solvents which are not miscible with one another.

## Revendications

1. Procédé de préparation de composés de formule générale

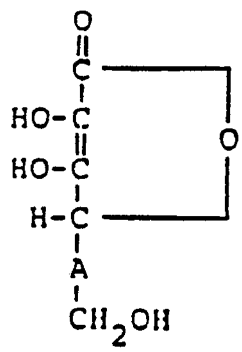

I

où A représente le groupe

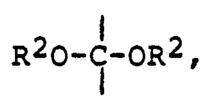, 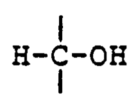

ou

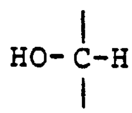

et $R^2$ représente un alcoyle en $C_1$ à $C_5$, caractérisé en ce qu'on fait réagir un composé de formule générale

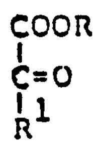

II

où R représente un alcoyle en $C_1$ à $C_5$ et $R^1$ un groupe de formule

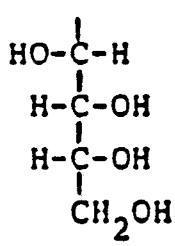,

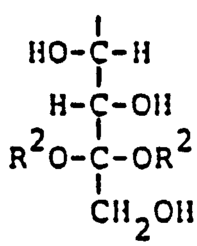

ou

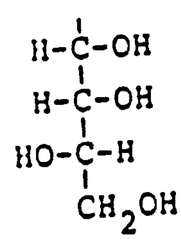

et $R^2$ a la signification donnée ci-dessus, avec une amine en $C_{12}$ à $C_{38}$ dans un solvant ou mélange de solvants organiques protiques ou aprotiques-dipolaires, en ce qu'on clive le sel d'amine obtenu de formule générale

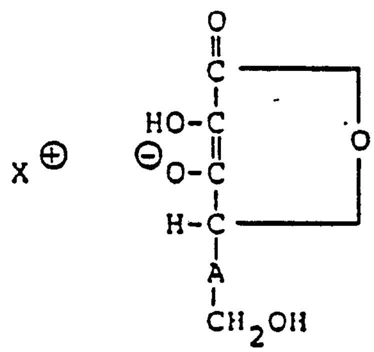

III

où A a la signification donnée ci-dessus et X⊕ représente l'ion ammonium de l'amine utilisée,

sans neutralisation par extraction liquide-liquide ou par digestion avec un solvant organique non-polaire et en ce qu'on isole le composé de formule I ainsi qu'éventuellement l'amine utilisée.

2. Procédé selon la revendication 1, caractérisé en ce qu'on utilise comme amine une alcoylamine tertiaire à chaîne droite en $C_{15}$ à $C_{30}$.

3. Procédé selon la revendication 1, caractérisé en ce qu'on utilise comme amine une alcoylamine secondaire ramifiée en $C_{16}$ à $C_{25}$.

4. Procédé selon la revendication 1, caractérisé en ce qu'on utilise comme amine une alcoylamine primaire ramifiée en $C_{12}$ à $C_{24}$.

5. Procédé selon la revendication 1 ou 2, caractérisé en ce qu'on utilise comme amine la tripentylamine, triisopentylamine, N,N-dioctylméthylamine, trihexylamine, triheptylamine, trioctylamine ou tridodécylamine.

6. Procédé selon la revendication 1 ou 3, caractérisé en ce qu'on utilise comme amine la Bis-(2-éthylhexyl)amine, dioctylamine, Amberlite LA-1® ou Amberlite LA-2®.

7. Procédé selon la revendication 1 ou 4, caractérisé en ce qu'on utilise comme amine Primene JMT® ou Amberlite LA-3®.

8. Procédé selon l'une des revendications 1 à 7, caractérisé en ce qu'on utilise l'amine en une quantité de 0,1 à 1,5 , de préférence de 0,5 à 1,1 mole par mole d'ester.

9. Procédé selon la revendication 8, caractérisé en ce qu'on utilise l'amine en une quantité de 0,9 à 1 mole par mole d'ester.

10. Procédé selon l'une des revendications 1 à 9, caractérisé en ce qu'on conduit le clivage du sel d'amine par extraction liquide-liquide au moyen de solvants non-polaires/polaires non miscibles entre eux.